# EUROPÄISCHE PATENTANMELDUNG

(11) **EP 3 552 601 A1**
(43) Veröffentlichungstag der Anmeldung: **16.10.2019**
(21) Anmeldenummer: 18166795.7
(22) Anmeldetag: 11.04.2018
(51) Int. Cl.: A61K 9/00, A61K 47/10, A61K 47/14, A61K 9/10, A61K 47/44

(54) **PHARMAZEUTISCHE ZUSAMMENSETZUNG ENTHALTEND DEXPANTHENOL UND POLIHEXANID**

(71) Anmelder: Bodenschatz, Karl, 90469 Nürnberg (DE)
(72) Erfinder: Bodenschatz, Karl, 90469 Nürnberg (DE)
(74) Vertreter: Dr. Gassner & Partner mbB

(57) **Zusammenfassung**

Die Erfindung betrifft eine pharmazeutische Zusammensetzung, enthaltend Dexpanthenol und Polihexanid zur Verwendung bei der topischen Behandlung einer Verletzung der Haut bei einem Menschen oder einem Tier, wobei die pharmazeutische Zusammensetzung als Emulsion mit einer lipophilen Phase und einer hydrophilen Phase formuliert ist, wobei die hydrophile Phase das Dexpanthenol und das Polihexanid enthält.

## Beschreibung

Die Erfindung betrifft eine pharmazeutische Zusammensetzung enthaltend Dexpanthenol und Polihexanid.

Aus der EP 1 992 340 B1 ist eine ophthalmische Lösung, welche Dexpanthenol, einen Phosphatpuffer und Polyhexamethylenbiguanid (= Polihexanid) enthält, bekannt. Unter ophthalmischen Lösungen werden Lösungen zur Behandlung von Kontaktlinsen sowie Benetzungslösungen und ins Auge zu gebende Lösungen zur Behandlung von Augenzuständen verstanden.

Aus der DE 20 2011 108 802 U1 ist eine Wundauflage zur therapeutischen Wundversorgung bekannt, wobei die Wundauflage mindestens eine wundabdeckende Schicht und mindestens ein Sorptionsmittel auf Basis einer Aktivkohle umfasst. Die Aktivkohle weist eine biozide und/oder biostatische Wirkung und/oder Ausrüstung auf. Dazu kann die Aktivkohle mit mindestens einem bioziden und/oder biostatischen Wirkstoff ausgerüstet sein. Der Wirkstoff kann aus der Gruppe von Polyhexamethylenbiguanid, Taurolidin, Benzalkoniumchlorid, Chlorhexidin, Octenidin und deren physiologisch verträglichen Salzen und Derivaten sowie deren Mischungen ausgewählt sein. Die Aktivkohle kann außerdem mit mindestens einem weiteren Wirkstoff ausgerüstet und/oder imprägniert sein. Der weitere Wirkstoff kann ein Wirkstoff mit wundheilungsfördernder Wirkung sein. Er kann aus der Gruppe von Alginaten, Chitosan, Hyaluronsäure und deren Salzen, Allantoin, beta-Sitosterol, Bromelain, Dexpanthenol, Pantothensäure, Harnstoff, Flavonoiden, Riboflavin, Saponinen, Cineol, Tocopherol und deren Mischungen ausgewählt sein.

Die EP 2 196 225 A2 offenbart eine Zubereitung, insbesondere Wundversorgungszubereitungen, umfassend ein oder mehrere filmbildende hydrophobe acrylatbasierende Polymere, Wasser, wasserlösliche Wirkstoffe und ein oder mehrere Lösevermittler gewählt aus der Gruppe Isopropylalkohol, Propylalkohol, und/oder 2-Phenoxyethanol. Die Wirkstoffe können gewählt werden aus der Gruppe der Antiseptika, insbesondere Benzalkoniumchlorid, Octenidin (Octenidindihydrochlorid), Chlorhexidin, Chlorhexidindigluconat, Chlorhexidindiacetat-Monohydrat und/oder Polihexanid, oder aus der Gruppe Dexpanthenol, Polidocanol, Hamamelis Extrakt, Magnolienextrakt und Pfingstrosenextrakt.

Aufgabe der vorliegenden Erfindung ist es, eine alternative pharmazeutische Zusammensetzung anzugeben, welche besonders gut zur Verwendung bei der topischen Behandlung einer Verletzung der Haut bei einem Menschen oder einem Tiere, insbesondere Säugetier, geeignet ist. Bei der Verletzung kann es sich um eine Brandverletzung, insbesondere eine verhältnismäßig großflächige Brandverletzung, handeln.

Die Aufgabe wird durch die Merkmale des Patentanspruchs 1 gelöst. Zweckmäßige Ausgestaltungen ergeben sich aus den Merkmalen der Patentansprüche 2 bis 13.

Erfindungsgemäß ist eine pharmazeutische Zusammensetzung enthaltend Dexpanthenol und Polihexanid zur Verwendung bei der topischen Behandlung einer Verletzung der Haut bei einem Menschen oder einem Tier, insbesondere einem Säugetier, vorgesehen. Die pharmazeutische Zusammensetzung ist dabei als Emulsion mit einer lipophilen Phase und einer hydrophilen Phase formuliert, wobei die hydrophile Phase das Dexpanthenol und das Polihexanid enthält. Die lipophile Phase kann dabei mindestens einen Bestandteil aus einer Gruppe, bestehend aus Caprinsäure, Caprylsäure, Capronsäure, Laurinsäure, Myristinsäure, Paraffin, Glycerolmonostearat, Wachs, insbesondere gebleichtes Wachs, Wollwachs, Wollwachsalkohol, Mandelöl, Sonnenblumenöl, Rizinusöl, Erdnussöl, Olivenöl, Mineralöl, 2-Ethylhexyllaurat, Decyloleat, Cetylstearylalkohol, insbesondere Cetylstearylalkohol Typ A und Vaselin, insbesondere weißes Vaselin, umfassen oder aus mindestens einem dieser Bestandteile bestehen. Die hydrophile Phase kann mindestens einen Bestandteil aus einer Gruppe, bestehend aus Wasser, einem Alkohol, Glycerin, Polyethylenglykol und/oder Propylenglycol, umfassen oder aus mindestens einem dieser Bestandteile bestehen. Weiterhin sind im Allgemeinen Emulgatoren, wie z. B. Glycerolmonostearat 60, Cetylalkohol, und/oder Macrogol-1000-glycerolmonostearat, enthalten. Der pH-Wert der Emulsion kann dabei im Bereich von 4 bis 7, insbesondere 4,5 bis 6,5, liegen.

Eine gut geeignete Emulsion ist beispielsweise die sogenannte Basiscreme DAC gemäß dem deutschen Arzneimittel-Codex (DAC), in welche Dexpanthenol und Polihexanid eingemischt ist. 100 g der Basiscreme DAC sind wie folgt zusammengesetzt: 4,0 g Glycerolmonostearat 60, 6,0 g Cetylalkohol, 7,5 g mittelkettige Triglyceride (Neutralöl, Miglyol 812), 25,5 g weißes Vaselin, 7,0 g Macrogol-1000-glycerolmonostearat, 10,0 g Propylenglycol und 40,0 g gereinigtes Wasser.

Der Erfinder hat erkannt, dass sich die erfindungsgemäße pharmazeutische Zusammensetzung besonders gut zur Behandlung von Verletzungen der Haut eignet, bei denen, insbesondere verhältnismäßig großflächig, neue Haut gebildet werden soll, um eine Hautersatzoperation, wie beispielsweise eine Eigenhauttransplantation, zu vermeiden oder ihren erforderlichen Umfang zu reduzieren. Die erfindungsgemäße pharmazeutische Zusammensetzung kann für mehrere Tage, beispielsweise unter einem Verband, auf der Verletzung der Haut bleiben, ohne dass eine erneute Wundbehandlung erforderlich ist. Ein Verbandswechselintervall kann dadurch gegenüber den bisher üblichen Intervallen deutlich verlängert werden. Das Polihexanid verhindert dabei eine Verkeimung der erfindungsgemäßen pharmazeutische Zusammensetzung sowie eine Infektion und eine damit einhergehende Entzündungsreaktion der verletzten Haut. Es ist mit der erfindungsgemäßen pharmazeutischen Zusammensetzung sogar gelungen, eine mit multiresistenten Keimen verkeimte Hautwunde zu entkeimen und zu sanieren. Das Dexpanthenol bewirkt in Kombination mit der Emulsion und dem Polihexanid eine schnelle Hautneubildung. Gleichzeitig hemmt das Dexpanthenol eine Proliferation von Fibroblasten des Bindegewebes unterhalb der neu zu bildenden Haut und somit eine Entstehung von überschüssigem Granulationsgewebe und eine Narbenbildung. Die lipophile Phase hat eine wundpflegende und wundberuhigende Wirkung.

Der Erfinder hat weiterhin erkannt, dass die Behandlung einer Verletzung der Haut bei einem Menschen oder einem Tier mit der erfindungsgemäßen pharmazeutischen Zusammensetzung zu einer deutlich schnelleren Heilung führt als eine abwechselnde Behandlung mit Polihexanid und Dexpanthenol. Dadurch kann ein Verbandswechsel deutlich seltener erfolgen als dies bisher üblich ist. Insbesondere bei sehr schmerzhaften Verletzungen der Haut, wie beispielsweise Brandverletzungen, kann dadurch auch die Anzahl der zum Verbandswechsel häufig erforderlichen Sedierungen vermindert werden. Damit können auch die mit häufigen Sedierungen einhergehenden Nebenwirkungen vermindert werden.

Bei einer Ausgestaltung der erfindungsgemäßen pharmazeutischen Zusammensetzung enthält die lipophile Phase mindestens ein Corticoid oder ein Salz, insbesondere ein physiologisch akzeptierbares Salz, eines Corticoids oder einen Ester, insbesondere einen physiologisch akzeptierbaren Ester, eines Corticoids. Insbesondere bei einem längeren Heilungsverlauf, vor allem bei einer verhältnismäßig großflächigen Verletzung der Haut, wirkt es sich günstig auf den Heilungsverlauf aus, wenn die lipophile Phase das mindestens eine Corticoid enthält. Durch das Corticoid kann eine Entzündungsreaktion unterdrückt oder zumindest vermindert werden. Eine Entzündungsreaktion würde sowohl noch vorhandenes als auch neugebildetes Hautgewebe zerstören und die Neubildung von Haut verhindern. Durch das Vorsehen einer Emulsion mit einer lipophilen Phase und einer hydrophilen Phase ist eine gleichzeitige Behandlung der Verletzung der Haut mit Dexpanthenol, Polihexanid und dem mindestens einen Corticoid oder Salz oder Ester davon möglich.

Bei einer mehr als 10% der Körperoberfläche betreffenden Verletzung der Haut erfolgt die topische Behandlung üblicherweise zunächst mit einer erfindungsgemäßen pharmazeutischen Zusammensetzung, deren lipophile Phase kein Corticoid, kein Salz und keinen Ester davon enthält. Nach einigen Tagen kann dann auf eine Behandlung mit der erfindungsgemäßen pharmazeutischen Zusammensetzung, deren lipophile Phase mindestens ein Corticoid oder ein Salz eines Corticoids oder einen Ester eines Corticoids enthält, umgestellt werden. Auch Verletzungen der Haut, welche infiziert sind, werden normalerweise mit einer erfindungsgemäßen pharmazeutischen Zusammensetzung behandelt, welche kein Corticoid, kein Salz eines Corticoids und keinen Ester eines Corticoids enthält. Bei Verletzungen der Haut, die bis zu 10% der Körperoberfläche betreffen und die nicht infiziert sind, wird die Behandlung üblicherweise mit der erfindungsgemäßen pharmazeutischen Zusammensetzung begonnen, deren lipophile Phase mindestens ein Corticoid, oder ein Salz eines Corticoids oder einen Ester eines Corticoids enthält.

Da üblicherweise nach etwa 3 bis 4 Wochen nicht durch Neubildung von Haut abgedeckte Bereiche der Verletzung durch Transplantation geschlossen werden sollten, um eine Infektion zu verhindern, können durch die mit der erfindungsgemäßen pharmazeutischen Zusammensetzung erreichbare Beschleunigung und Verbesserung des Heilungsprozesses Hauttransplantationen ganz vermieden werden oder zumindest kleinflächiger erfolgen. Dies gilt insbesondere dann, wenn die pharmazeutischen Zusammensetzung mindestens ein Corticoid oder Salz oder Ester davon enthält.

Bei dem Corticoid kann es sich um Cortison, Corticosteron, Cortisol, Aldosteron, Desoxycorticosteron, Prednison, Methylprednisolon, Triamcinolon, Betamethason, Paramethason, Hydrocortison, Hydrocortisonacetat, Hydrocortison-17-butyrat, Triamcinolonacetonid, Prednicarbat, Mometasonfuroat, Betamethason-17-valerat, Betamethasondipropionat, Clobetasol-17-propionat, Prednisolon, Prednisolon-21-acetat, Dexamethason, Dexamethason-21-acetat, Fluocinolonacetonid, Methylprednisolonaceponat, Desonid, Budesonid, Halcinoid, Desoximethason, Clocortolon, Fluticason, Fluocinoid, Halcinoid, Amcinonid, Diflorasondiacetat, Flurandrenolid oder Alclomethasondipropionat handeln.

Bei einer Ausgestaltung der erfindungsgemäßen pharmazeutischen Zusammensetzung ist das Corticoid oder das Salz des Corticoids oder der Ester des Corticoids darin in einer Konzentration von 0,001 bis 2,5 Gew.-%, insbesondere 0,005 bis 2 Gew.-%, enthalten. Alternativ kann das jeweilige Corticoid in der pharmazeutischen Zusammensetzung in den folgenden für das jeweilige Corticoid angegebenen Konzentrationen enthalten sein: Hydrocortison 0,1 bis 2 Gew.-% , Hydrocortisonacetat 0,1 bis 2 Gew.-%, Hydrocortison-17-butyrat 0,02 bis 0,2 Gew.-%, Triamcinolonacetonid 0,01 bis 0,2 Gew.-%, Prednicarbat 0,05 bis 0,5 Gew.-%, Mometasonfuroat 0,02 bis 0,2 Gew.-%, Betamethason-17-valerat 0,02 bis 0,2 Gew.-%, Betamethasondipropionat 0,01 bis 0,1 Gew.-%, Clobetasol-17-propionat 0,01 bis 0,1 Gew.-%, Prednisolon 0,05 bis 1 Gew.-%, Prednisolon-21-acetat 0,05 bis 1 Gew.-%, Dexamethason 0,005 bis 0,1 Gew.-%, Dexamethason-21-acetat 0,005 bis 0,1 Gew.-%, Fluocinolonacetonid 0,01 bis 0,1 Gew.-%, Methylprednisolonaceponat 0,02 bis 0,2 Gew.-%, Desonid 0,01 bis 0,1 Gew.-%, Budesonid 0,005 bis 0,05 Gew.-%, Halcinoid 0,01 bis 0,1 Gew.-%, Desoximethason 0,01 bis 0,25 Gew.-%, Clocortolon 0,01 bis 0,2 Gew.-%, Fluticason 0,01 bis 0,2 Gew.-%, Fluocinoid 0,01 bis 0,1 Gew.-%, Halcinoid 0,01 bis 0,1 Gew.-%, Amcinonid 0,01 bis 0,1 Gew.-%, Diflorasondiacetat 0,01 bis 0,1 Gew.-%, Flurandrenolid 0,01 bis 0,1 Gew.-% und Alclomethasondipropionat 0,01 bis 0,2 Gew.-%, insbesondere 0,04 bis 0,06 Gew.-%. "Gew.-%" ist im gesamten Text stets auf das Gesamtgewicht der erfindungsgemäßen pharmazeutischen Zusammensetzung bezogen.

In klinischen Versuchen hat es sich als günstig erwiesen, wenn Dexpanthenol in der erfindungsgemäßen pharmazeutischen Zusammensetzung in einer Konzentration von 1 bis 10 Gew.%, insbesondere 1,5 bis 5,5 Gew.-%, und/oder Polihexanid in der erfindungsgemäßen pharmazeutischen Zusammensetzung in einer Konzentration von 0,005 bis 0,3 Gew.%, insbesondere 0,01 bis 0,2 Gew.-%, enthalten sind/ist.

Die erfindungsgemäße pharmazeutische Zusammensetzung kann als Creme oder Lotion formuliert sein. Zur Behandlung der Verletzung kann die Creme oder Lotion direkt auf die verletzte Stelle der Haut aufgetragen werden. Ist auch möglich, eine Wundauflage mit der Creme zu bestreichen oder der Lotion zu tränken und diese dann auf die verletzte Stelle der Haut aufzubringen und gegebenenfalls mit einem Verband zu sichern.

Wenn die pharmazeutische Zusammensetzung als Creme formuliert ist, kann diese Glycerolmonostearat 60, Cetylalkohol, mindestens ein Triglycerid, insbesondere ein mittelkettiges Triglycerid, Vaselin, insbesondere weißes Vaselin, Macrogol-1000-glycerolmonostearat, Propylenglycol und Wasser enthalten. Mittelkettige Triglyceride sind Triglyceride deren Fettsäuren ein aliphatischen Kette von 6 bis 12 Kohlenstoff-Atomen aufweisen, beispielsweise Capronsäure (C₆H₁₂O₂), Caprylsäure (C₈H₁₆O₂), Caprinsäure (C₁₀H₂₀O₂) oder Laurinsäure (C₁₂H₂₄O₂). Ein hier als mittelkettige Triglyceride geeignetes Gemisch kann aus Caprinsäure und Caprylsäure bestehen. Ein solches Gemisch wird als Neutralöl bezeichnet und beispielsweise unter dem Namen Miglyol 812 vertrieben. Glycerolmonostearat 60, Cetylalkohol und Macrogol-1000-glycerolmonostearat dienen als Emulgatoren. Die Grundlage der genannten Creme kann die oben genannte Basiscreme DAC sein.

Bei der Verletzung der Haut kann es sich um eine Brandverletzung handeln. Die erfindungsgemäße pharmazeutische Zusammensetzung hat sich bei Brandverletzungen, insbesondere bei Brandverletzungen von Kindern, als besonders gut dazu geeignet erwiesen, eine verhältnismäßig schnelle Neubildung von Haut zu bewirken. Die Brandverletzung kann dabei eine Verbrennung zweiten oder dritten Grades sein.

Die Behandlung kann je nach Bedarf mindestens ein-, zwei- oder dreimal pro Woche, insbesondere mindestens einmal täglich, insbesondere zweimal täglich, erfolgen. Die mit der erfindungsgemäßen pharmazeutischen Zusammensetzung erreichbaren langen Behandlungsintervalle erlauben es aber auch, dass die Behandlung höchstens ein-, zwei- oder dreimal pro Woche erfolgt.

Nachfolgend wird die Erfindung anhand von Ausführungsbeispielen näher erläutert.

### Beispiel 1:

Eine erste erfindungsgemäße pharmazeutische Zusammensetzung wurde nach folgender Rezeptur hergestellt:
Hydrocortisonacetat: 2,0 g
Polihexanidlösung 20%: 0,8 g
Dexpanthenol: 20 g
Basiscreme DAC: ad 400,0 g

Zur Herstellung wurden 50 g der Basiscreme DAC in einem Mörser vorgelegt und die Wirkstoffe gemäß obiger Tabelle zugewogen. Anschließend wurde bei Raumtemperatur 3 min lang mit einem Pistill gerührt bis eine homogene Grundlage entstanden ist. Daraufhin wurde mit Basiscreme DAC auf 400 g aufgefüllt und 3 min mit einem Pistill gerührt bis eine homogene Creme entstanden ist. Die so erhaltene Creme enthielt 5 mg/g Hydrocortisonacetat, 0,4 mg/g Polihexanid und 50 mg/g Dexpanthenol.

### Beispiel 2:

Eine weitere erfindungsgemäße pharmazeutische Zusammensetzung wurde nach folgender Rezeptur hergestellt:
Polihexanidlösung 20%: 0,8 g
Dexpanthenol: 20 g
Basiscreme DAC: ad 400,0 g

Zur Herstellung wurden 50 g der Basiscreme DAC in einem Mörser vorgelegt und die Wirkstoffe gemäß obiger Tabelle zugewogen. Anschließend wurde bei Raumtemperatur 3 min lang mit einem Pistill gerührt bis eine homogene Grundlage entstanden ist. Daraufhin wurde mit Basiscreme DAC auf 400 g aufgefüllt und 3 min mit einem Pistill gerührt bis eine homogene Creme entstanden ist. Die so erhaltene Creme enthielt 0,4 mg/g Polihexanid und 50 mg/g Dexpanthenol.

Der mit den erfindungsgemäßen pharmazeutischen Zusammensetzungen gemäß Beispiel 1 und 2 behandelte Patient war ein zweijähriges Kind mit Verbrühungen zweiten und dritten Grades durch Wasser an Thorax, Bauch, Schulter, Oberarm und Unterarm sowie zirkulär am Handgelenk und dorsal an der rechten Hand und am rechten Mittelfinger. Insgesamt waren etwa 7% der Körperoberfläche betroffen. Drei Tage nach der Verbrühung wurden die großflächigen Verbrühungen mit einem synthetischen Hautersatzmaterial (Suprathel® der Firma PolyMedics Innovations GmbH, 73770 Denkendorf, Deutschland) abgedeckt. Nach zwei Tagen wurde das Hautersatzmaterial entfernt und die Verbrühungen mit der pharmazeutischen Zusammensetzung gemäß Beispiel 1 durch Auftragen auf die betroffenen Areale zweimal täglich behandelt. Sieben Tage später wurde die Behandlung auf eine pharmazeutische Zusammensetzung gemäß Beispiel 2 umgestellt, indem die pharmazeutische Zusammensetzung gemäß Beispiel 2 zweimal täglich auf die betroffenen Areale aufgetragen wurde. Nach drei Tagen wurden die betroffenen Areale dann bis zu einer vollständigen Heilung nur noch mit einer Dexpanthenol enthaltenden pharmazeutischen Zusammensetzung behandelt. Durch die Behandlung mit den erfindungsgemäßen pharmazeutischen Zusammensetzungen konnte eine verhältnismäßig kurze Zeit bis zur Heilung, eine verhältnismäßig geringe Narbenbildung und eine gute Heilung der betroffenen Areale erreicht werden.

## Patentansprüche

1. Pharmazeutische Zusammensetzung enthaltend Dexpanthenol und Polihexanid zur Verwendung bei der topischen Behandlung einer Verletzung der Haut bei einem Menschen oder einem Tier, wobei die pharmazeutische Zusammensetzung als Emulsion mit einer lipophilen Phase und einer hydrophilen Phase formuliert ist, wobei die hydrophile Phase das Dexpanthenol und das Polihexanid enthält.

2. Pharmazeutische Zusammensetzung nach Anspruch 1 zur Verwendung nach Anspruch 1, wobei die lipophile Phase mindestens ein Corticoid oder ein Salz eines Corticoids oder einen Ester eines Corticoids enthält.

3. Pharmazeutische Zusammensetzung nach Anspruch 2 zur Verwendung nach Anspruch 1, wobei das Corticoid Cortison, Corticosteron, Cortisol, Aldosteron, Desoxycorticosteron, Prednison, Methylprednisolon, Triamcinolon, Betamethason, Paramethason, Hydrocortison, Hydrocortisonacetat, Hydrocortison-17-butyrat, Triamcinolonacetonid, Prednicarbat, Mometasonfuroat, Betamethason-17-valerat, Betamethasondipropionat, Clobetasol-17-propionat, Prednisolon, Prednisolon-21-acetat, Dexamethason, Dexamethason-21-acetat, Fluocinolonacetonid, Methylprednisolonaceponat, Desonid, Budesonid, Halcinoid, Desoximethason, Clocortolon, Fluticason, Fluocinoid, Halcinoid, Amcinonid, Diflorasondiacetat, Flurandrenolid oder Alclomethasondipropionat ist.

4. Pharmazeutische Zusammensetzung nach Anspruch 2 oder 3 zur Verwendung nach Anspruch 1, wobei das Corticoid oder das Salz des Corticoids oder der Ester des Corticoids darin in einer Konzentration von 0,001 bis 2,5 Gew.-%, insbesondere 0,005 bis 2 Gew.-%, oder das jeweilige Corticoid darin in den folgenden für das jeweilige Corticoid angegebenen Konzentrationen enthalten ist: Hydrocortison 0,1 bis 2 Gew.-% , Hydrocortisonacetat 0,1 bis 2 Gew.-%, Hydrocortison-17-butyrat 0,02 bis 0,2 Gew.-%, Triamcinolonacetonid 0,01 bis 0,2 Gew.-%, Prednicarbat 0,05 bis 0,5 Gew.-%, Mometasonfuroat 0,02 bis 0,2 Gew.-%, Betamethason-17-valerat 0,02 bis 0,2 Gew.-%, Betamethasondipropionat 0,01 bis 0,1 Gew.-%, Clobetasol-17-propionat 0,01 bis 0,1 Gew.-%, Prednisolon 0,05 bis 1 Gew.-%, Prednisolon-21-acetat 0,05 bis 1 Gew.-%, Dexamethason 0,005 bis 0,1 Gew.-%, Dexamethason-21-acetat 0,005 bis 0,1 Gew.-%, Fluocinolonacetonid 0,01 bis 0,1 Gew.-%, Methylprednisolonaceponat 0,02 bis 0,2 Gew.-%, Desonid 0,01 bis 0,1 Gew.-%, Budesonid 0,005 bis 0,05 Gew.-%, Halcinoid 0,01 bis 0,1 Gew.-%, Desoximethason 0,01 bis 0,25 Gew.-%, Clocortolon 0,01 bis 0,2 Gew.-%, Fluticason 0,01 bis 0,2 Gew.-%, Fluocinoid 0,01 bis 0,1 Gew.-%, Halcinoid 0,01 bis 0,1 Gew.-%, Amcinonid 0,01 bis 0,1 Gew.-%, Diflorasondiacetat 0,01 bis 0,1 Gew.-%, Flurandrenolid 0,01 bis 0,1 Gew.-% und Alclomethasondipropionat 0,01 bis 0,2 Gew.-%.

5. Pharmazeutische Zusammensetzung nach einem der vorherigen Ansprüche zur Verwendung nach Anspruch 1, wobei die lipophile Phase mindestens einen Bestandteil aus einer Gruppe, bestehend aus Caprinsäure, Caprylsäure, Capronsäure, Laurinsäure, Myristinsäure, Paraffin, Glycerolmonostearat, Wachs, Wollwachs, Wollwachsalkohol, Mandelöl, Sonnenblumenöl, Rizinusöl, Erdnussöl, Olivenöl, Mineralöl, 2-Ethylhexyllaurat, Decyloleat, Cetylstearylalkohol und Vaselin, umfasst oder aus mindestens einem dieser Bestandteile besteht.

6. Pharmazeutische Zusammensetzung nach einem der vorherigen Ansprüche zur Verwendung nach Anspruch 1, wobei die hydrophile Phase mindestens einen Bestandteil aus einer Gruppe, bestehend aus Wasser, einem Alkohol, Glycerin, Polyethylenglykol und Propylenglycol, umfasst oder aus mindestens einem dieser Bestandteile besteht.

7. Pharmazeutische Zusammensetzung nach einem der vorherigen Ansprüche zur Verwendung nach Anspruch 1, wobei Dexpanthenol darin in einer Konzentration von 1 bis 10 Gew.%, insbesondere 1,5 bis 5,5 Gew.-%, und/oder Polihexanid darin in einer Konzentration von 0,005 bis 0,3 Gew.%, insbesondere 0,01 bis 0,2 Gew.-%, enthalten sind/ist.

8. Pharmazeutische Zusammensetzung nach einem der vorherigen Ansprüche zur Verwendung nach Anspruch 1, wobei diese als Creme oder Lotion formuliert ist.

9. Pharmazeutische Zusammensetzung nach einem der vorherigen Ansprüche zur Verwendung nach Anspruch 1, wobei diese als Creme formuliert ist, die Glycerolmonostearat 60, Cetylalkohol, mindestens ein Triglycerid, Vaselin, Macrogol-1000-glycerolmonostearat, Propylenglycol und Wasser enthält.

10. Pharmazeutische Zusammensetzung nach einem der vorherigen Ansprüche zur Verwendung nach Anspruch 1, wobei die Verletzung der Haut eine Brandverletzung ist.

11. Pharmazeutische Zusammensetzung nach einem der vorherigen Ansprüche zur Verwendung nach Anspruch 1, wobei die Brandverletzung eine Verbrennung zweiten oder dritten Grades ist.

12. Pharmazeutische Zusammensetzung nach einem der vorherigen Ansprüche zur Verwendung nach Anspruch 1, wobei die Behandlung mindestens ein-, zwei- oder dreimal pro Woche, insbesondere mindestens einmal täglich, erfolgt.

13. Pharmazeutische Zusammensetzung nach einem der vorherigen Ansprüche zur Verwendung nach Anspruch 1, wobei die Behandlung höchstens ein-, zwei- oder dreimal pro Woche erfolgt.
